# EUROPEAN PATENT SPECIFICATION

(11) **EP 0 552 518 B1**
(45) Date of publication and mention of the grant of the patent: **13.09.1995**
(21) Application number: 92300311.5
(22) Date of filing: 14.01.1992
(51) Int. Cl.: C07C 37/86, C07C 39/16

(54) **Method of recovering bisphenol-A from preparative process efluent streams**
Verfahren zur Wiedergewinnung von Bisphenol-A aus Ausflusströmen des Herstellungsverfahrens
Procédé de récupération de bisphénol-A à partir des écoulements du procédé de fabrication

(43) Date of publication of application: 28.07.1993
(73) Proprietor: GENERAL ELECTRIC COMPANY, Schenectady, NY 12345 (US)
(72) Inventor: Johnson, Philip C., Morgantown, West Virginia 26505 (US); Cipullo, Michael John, Mount Vernon, Indiana 47620 (US); Johnson, Norman Enoch, Pittsfield, Massachusetts 01201 (US)
(74) Representative: Pratt, Richard Wilson

(56) References cited:
- US-A- 3 242 220
- US-A- 4 354 046
- US-A- 4 375 567
- US-A- 4 766 254

## Description

### BACKGROUND OF THE INVENTION

### Field of the Invention

The invention relates to processes for the synthesis of dihydric phenols and more particularly to a process for recovering bisphenol-A.

### Brief Description of Related Art

The dihydric phenol 2,2 bis(p-hydroxyphenyl) propane (commonly referred to as "bisphenol-A") is commercially prepared by condensing 2 moles of phenol with a mole of acetone in the presence of an acid catalyst. The phenol is present in a molar excess of the stoichiometric requirement. During the condensation, a number of by-products are formed which are contaminants of the desired product, bisphenol-A. These contaminants, carried in the product stream from the condensation reaction zone, include water, trace quantities of acidic materials derived from the catalyst, unreacted phenol and acetone and a number of isomers of bisphenol-A.

Conventionally, a first step in separating the desired bisphenol-A from the product stream may involve cooling the product stream to induce crystallization and precipitation of the bisphenol-A in the form of a 1:1 adduct with the excess of phenol present. The crystallized adduct is separated, washed, and the phenol removed by distillation, extraction or steam stripping. In this separation, the mother liquor obtained following crystallization contains further quantities of bisphenol-A in admixture with the previously described contaminants. Other streams are frequently generated carrying such or similar residues, during the various bisphenol-A purification procedures. While many of these streams are often recycled to the reactor for further condensation reaction, portions must be purged or "cracked" back to phenol, acetone or isopropyl phenol to recover valuable chemicals.

The method of the present invention provides an economical means to recover bisphenol-A and other valuable residues from admixture with contaminant materials in the preparative process effluent streams, without employing cracking procedures. Such cracking procedures would convert bisphenol-A back to phenol and acetone.

The use of anionic exchange resins to remove acidic contaminants from phenol-containing process streams has been described previously. For example, European Patent Application 0 329 075 published August 23, 1989 (Mitsui Toatsu Chemicals, Inc.) describes bisphenol-A product streams containing residual phenol reactant and acid contaminants. The product stream is treated with weakly basic ion-exchange resins to remove the acid contaminants before separating the desired bisphenol-A by distillation. A similar treatment of bisphenol-A product streams is described in U.S. Patent 4,766,254 (Faler et al.) wherein mother liquor from a phenol-bisphenol-A adduct is treated with an anion exchange resin.

US-A-4375567 discloses a method for making bisphenol-A, based on the condensation of acetone and phenol in the presence of an ion-exchange resin. Improved yields of 2,2-bis(4-hydroxyphenyl)propane are achieved by recycling bis-phenol-A reaction mixture containing significant amounts of 2,4'-dihydroxy-2,2-diphenyl propane and the use of a macroreticular ion-exchange resin in the isomerisation zone.

### SUMMARY OF THE INVENTION

The invention comprises, a method of recovering bisphenol-A from a mother liquor obtained upon crystallization of a 1:1 adduct of phenol and bisphenol-A, said mother liquor containing phenol, bisphenol-A, isomers of bisphenol-A and contaminant by-products of the condensation reaction of phenol with acetone, which comprises;
A. isomerizing isomers of bisphenol-A to bisphenol-A;
B. removing acidic impurities from the resulting mixture by reaction with a basic ion exchange resin; and
C. separating bisphenol-A from the acid free isomerate.

Additionally, the process recovers bisphenol-A value from any process stream from which the bulk of bisphenol-A has been previously removed.

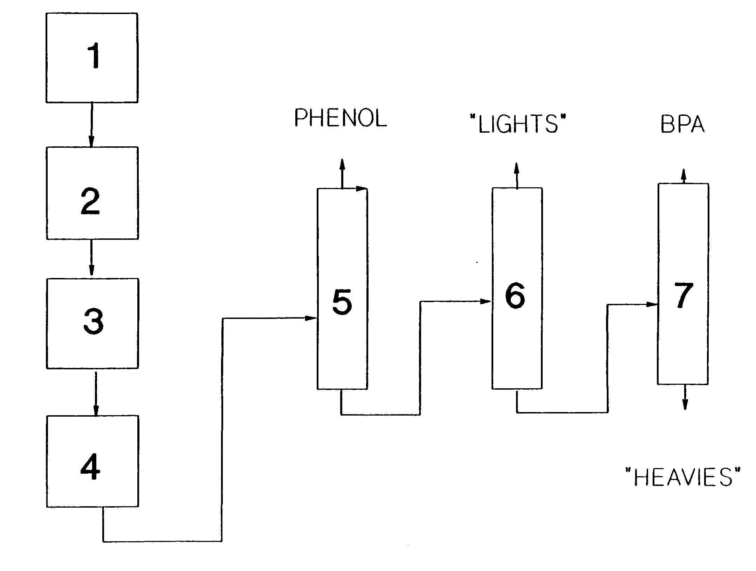

### BRIEF DESCRIPTION OF THE DRAWING

The drawing is a block diagram representing a preferred embodiment of the invention.

### DETAILED DESCRIPTION OF THE PREFERRED EMBODIMENT OF THE INVENTION

The commercially important processes for preparing bisphenol-A comprise condensation of 2 moles of phenol with a mole of acetone in the presence of an acid catalyst and a stoichiometric excess of the phenol reactant; see for example the U.S. Patents 4,766,254 and 4,847,433. One catalyst frequently used is an acidic ion-exchange catalyst.

Acidic ion-exchange resins useful to catalyze the condensation of phenol with acetone are generally well known compositions as are methods of their preparation; see for example the preparative procedures described in U.S. Patent 3,037,052 which is hereby incorporated herein by reference thereto. Representative of acid ion-exchange resins are strong-acid ion exchangers, such as those resins or polymers having a plurality of pendant sulfonic acid groups. Examples include sulphonated polystyrene or poly(styrene-divinylbenzene) copolymer and sulphonated phenol-formaldehyde resins. The sulphonated resins are commercially available in water swollen form as gellular and macro-reticular types. Specific examples of commercially available resins are Amberlite IR-120H, Amberlyst 15H, Amberlyst 31, Dowex 50-X-4, Dowex MSC-1H, Duolite c-26, Permutit QH, Chempro C-2 and Imac C8P/H (Amberlite, Amberlyst, Dowex, Duolite, Permutit, Chempro and Imac are registered U.S. Trademarks). Further examples of such ion exchangers as well as methods for preparing such ion exchangers are described in the Encyclopedia of Polymer Science and Technology; 1967, vol. 7, pages 695 to 708 and third edition, 1981. The exchange capacity of the acidic resin is preferably at least 2.0 meq. H⁺/g of dry resin, with exchange capacities in the range of from 3.0 to 5.5 meq. H⁺/g (dry resin) particularly preferred. One preferred catalyst is the Amberlyst® gelular types, which are styrene cross-linked with divinylbenzene or like cross-linking monomer and having pendant sulfonic acid groups attached to the aromatic nucleus of the styrene moiety. Sulfonation may be by the process described in U.S. Patent 2,366,007 which is incorporated herein by reference thereto.

Advantageously, the catalyst is employed initially in a substantially dry (dewatered) form, for maximum efficiency (recognizing that water is a by-product of the condensation reaction).

The condensation reaction zone may comprise a single reactor or two or more reactors in series or in parallel. In the case of a multi-reactor reaction zone, suitably all of the phenol is fed to the first reactor and the acetone compound is either fed all to the first reactor or divided between the first and second and possibly further reactors.

The reaction temperature in the reactor zone may vary from 40° C. to 95° C. with reaction temperatures in the range of from 55° C. to 90° C. being preferred.

The reaction time in the reactor zone may vary and depends on reaction temperature. For example, the liquid hour space velocity (LHSV) of the feed may vary between wide limits with velocities in the range of from 0.2 to 40 liters feedstream/liter catalyst⁻¹/hour⁻¹.

The reaction zone effluent is continuously withdrawn and fed to a system for separation of the product bisphenol-A. As mentioned earlier, this effluent comprises unreacted phenol, unreacted acetone, acid residues of the catalyst, water and isomers of bisphenol-A in admixture with the desired bisphenol-A. The isomers of interest are position isomers wherein the hydroxy groups are other than in the p-p'configuration. It is this effluent which may provide a starting material for the method of the present invention. The effluent may be treated first by cooling to precipitate a crystalline 1:1 adduct of bisphenol-A with phenol, and separating the solid adduct. The remaining mother liquor generally contains appreciable quantities of residual bisphenol-A.

The accompanying drawing is a block diagram showing an embodiment process of the invention, beginning with the mother liquor described above.

At least a portion of the mother liquor is fed from a storage tank (1) to an "isomerization" reactor (2) containing a strongly acidic ion exchange resin, such as one selected from those described above, conventionally used as an acid catalyst to catalyze the condensation of phenol with acetone. The acid catalyst is present in a catalytic proportion to promote isomerization of the bisphenol-A isomers. Interestingly not only are geometric isomers of bisphenol-A converted to bisphenol-A but also nonisomeric but structually analagous compounds such as 4,4'-(4-hydroxy-m-phenylene diisopropylidene) diphenol, otherwise known as "trisphenol" or "BPX-I" are also converted to bisphenol-A.

In the isomerization reactor many of the by-product isomers of bisphenol-A such as 2,4'-(1-methylethylidene) bisphenol and the like and some compounds with unknown structures are continuously converted to bisphenol-A. The mother liquor contents of the reactor (2) are heated to a temperature within the range of from about 55°C. to 95°C. to promote the isomerization. Progress of the conversion may be followed by conventional analytical technique to observe the conversion of the isomers.

When complete, the effluent of the reactor (2) passes through a vessel (3) containing a weakly basic ion exchange resin which removes acidic impurities, usually in trace quantities.

The ion exchange resins useful in this step of the invention include all known basic resins. For the most part, they are amine or quaternary ammonium resins typically containing such moieties as dimethylbenzylamino groups or corresponding methylquaternized groups attached to a polymer chain. Amine resins including those having a pyridyl group are often preferred. For the purpose of the invention, the amine resins are employed in the free base form, although quaternary resins wherein the counterion therein is a hydroxide anion can also be used.

Methods of preparing anionic exchange resins are generally well known; see for example the method described in U.S. Patent 2,632,001 (McMasters et al.) incorporated herein by reference thereto. This method comprises the side-chain chlorination of poly(vinyltoluene), followed by reaction with a tertiary amine in the presence of a polar solvent such as water to form a quaternary ammonium salt. Representative of commercially available resins of this type are Amberlite IRA-400, Amberlite IRA-401, Amberlite IRA-402, Amberlite IRA-900, Duolite A-101-D. Duolite ES-111, Dowex 1, Dowex 11, Dowex 21K, and Ionac A-540 and those derived from dimethylethanolamine CH₃)₂-NCH₂CH₂OH, Amberlite IRA-410, Amberlite IRA-911, Dowex 2, Duolite A-102-D, Ionac A-542, Ionac A-550 and Reillex (Amberlite, Duolite, Dowex and Ionac are registered U.S. trademarks).

Preferred for use in the present invention are weak-base anionic exchange resins, containing primary, secondary and tertiary amine groups. Commercially available examples of these products are Amberlite A-21, Dowex 44, Duolite A-7, Ionac A-260, Amberlite IRA-35, Amberlite IRA-68 (the latter two resins are gelular with acrylic backbones).

Contact between the reactor (2) effluent mixture and the anion exchange resin may be effected by any convenient means. It is generally preferred to pass the mixture for purification through a column containing the resin, at temperatures in the range of about 45°-125° C. and preferably about 50°-75° C. Passage of the phenol mixture through the column may be upward or downward, for a time sufficient to remove the acidic contaminants.

Upon contact with the anion exchange resin, acidic impurities are removed by salt formation therewith.

The treatment of the effluent mixture from reactor (2) with the weakly basic ion-exchange resin may be carried out continuously or batchwise. Generally, the lower the flow rate, the greater the effective efficiency of the removal of the acidic contaminants. For continuous treatment, the effluent mixture is preferably fed at a flow rate of 2 to 1000 kg/hr for 1 kg of the weakly basic ion-exchange resin, preferably 3-50. For batchwise treatment, the effluent mixture is preferably mixed with 1 to 20 wt% of the weakly basic ion-exchange resin, for 5 to 60 minutes.

When the resin has been exhausted it may be regenerated, for example by treatment with a 6 to 10 percent aqueous caustic. Such treatment can be preceded by washing with liquid phenol, to remove phenol soluble impurities whose presence would decrease the efficiency of the resin or regenerated, followed by a water wash.

The stream effluent from the vessel (3) then passes through a filter (4) which removes particulates (eg. ion exchange resin particles, metal fragments, etc.). The filter (4) will remove particles having a size greater than about 5 microns. A series of distillation columns (5, 6, and 7) follow, each of which operate under progressively (sequentially) higher vacuum/temperature conditions than the preceding column in order to separate the stream into four fractions. Distillation column (5) is operated under vacuum and temperature conditions in a manner to remove an overhead fraction containing relatively pure phenol (eg >97% purity). The bottoms of column (5) are fed to distillation column (6). This column operates under reduced pressures and elevated temperature conditions which remove a colored "light" fraction consisting mainly of residual isomers of bisphenol-A and chroman along with other byproducts with known and unknown chemical structure. This fraction can be purged from the plant and discarded, or subjected to additional recovery by this or other processes. The bottoms of column (6) are fed to column (7). The overheads of this column typically contain 60-90% pure bisphenol-A depending on the temperature and pressure conditions used. The bottoms of the column (7) contain "heavies" which are purged from the process. These heavies can be subjected to further recovery processes (eg. returned to another location in the bisphenol-A manufacturing process, cracked back to phenols or distilled further, burned, or used in other applications).

The following example and preparations describe the manner and process of making and using the invention and set forth the best mode contemplated by the inventor of carrying out the invention but are not to be construed as limiting the invention.

With reference to the apparatus described above, a bisphenol-A process by-product stream containing approximately 83.3% phenol, 9.8% bisphenol-A, 2.3% 2-(4-hydroxyphenyl)2-(2-hydroxyphenyl) propane (o, p bisphenol-A) and 4.6% other by-products was continually passed from a storage tank (1) through a jacketed "isomerization" reactor (2) containing a bed of sulfonated polystyrene resin (Rhom & Haas Amberlyst 31; 7483 kg (16500 lbs) of catalyst-dry basis) at a temperature of 74°C. and a flow of 20 gpm. At least a portion of the reaction effluent (82.5% phenol, 11.8% bisphenol-A, 1.6% 2-(4-hydroxyphenyl)2-(2-hydroxyphenyl) propane and 4.1% other by-products) was then passed through vessel (3) at a temperature of 72°C. containing 590 kg (1300 lbs) (dry basis) of Rhom & Haas Amberlyst A-21 weakly basic anionic resin to reduce the concentration of acidic species to <2ppm. Passing the resulting effluent solution through a filter (4) removed particulates larger than 2 microns.

At least a portion of the resulting solution was then fed to a phenol distillation column (5) which operated at a pressure of 40 mbar (30mm of mercury)and a temperature of 218°C. This removed the phenol as an approximately 99% pure colorless overhead material. The bottoms contained 1% phenol and were continuously fed to the next distillation column (6) which operated at a temperature of 224°C. and approximately 1.33-2.66 mbar (1-2mm of Hg). The overhead stream from this column was amber in color and consisted mainly of bisphenol-A isomers, primarily o, p-bisphenol-A (45-50%), 2,2,4-trimethyl-4(4-hydroxyphenol) Chroman (30-35%), bisphenol-A (2%) and other by-products. This stream was discarded.

The bottoms of column (6) was fed to a distillation column (7) which operated at a temperature of 260°C to 290°C. (500-550F) and a pressure of 1.33-2.66 mbar (1-2 mm of Hg). The column overheads consisted of 80+% pure slightly colored bisphenol-A which was recycled back to the bisphenol-A manufacturing process. The bottoms of the column contained a highly colored tar (<30% bisphenol-A, >70% heavy phenolic process by-products) which was discarded.

This process provides an efficient, low cost method of recovering bisphenol-A and other materials of value from process streams which would be normally recycled to the initial condensation reactor, cracked down to phenols or disposed of by burning.

## Claims

1. A method of recovering bisphenol-A from a mother liquor obtained upon crystallization of a 1:1 adduct of phenol and bisphenol-A, said mother liquor containing phenol, bisphenol-A, isomers of bisphenol-A, contaminant by-products of the condensation reaction of phenol with acetone and acidic impurities, which comprises;
(A.) contacting at least a portion of said mother liquor with a catalyst which isomerizes isomers of bisphenol-A to bisphenol-A;
(B.) contacting at least a portion of the resultant effluent of (A.) with a basic ion exchange resin, thereby removing acidic impurities; and
(C.) separating bisphenol-A from the acid free effluent of (B.)

2. The method of claim 1 wherein particulates are removed from the acid free material by filtration.

3. The method of claim 1 wherein separating is by distillation.

4. The method of claim 3 wherein distillation removes, in sequence, (a) phenol, (b) colored light fraction and (c) bisphenol-A.

5. A method of recovering bisphenol-A from a mixture of bisphenol-A, isomers of bisphenol-A, phenol, acidic impurities and contaminant by-products of the condensation reaction of acetone with phenol, which comprises;
A. isomerizing the isomers of bisphenol-A to bisphenol-A;
B. removing the acid contaminants from the isomerate;
C. filtering the acid free isomerate to remove solid particulates;
D. distilling the particulate free material to remove phenol;
E. distilling the bottoms of (D.) to remove colored light fraction; and
F. distilling the bottoms of (E.) to separate the bisphenol-A.

## Patentansprüche

1. Verfahren zur Rückgewinnung von Bisphenol-A aus einer Mutterlauge, die nach der Kristallisation eines 1:1 Aduktes aus Phenol und Bisphenol-A erhalten worden ist, wobei besagte Mutterlauge enthält: Phenol, Bisphenol-A, Isomere des Bisphenol-A, verunreinigende Nebenprodukte der Kondensationsreaktion von Phenol mit Aceton und Säureverunreinigungen, welches umfaßt:
(A) Inkontaktbringen von wenigstens einem Teil der besagten Mutterlauge mit einem Katalysator, der Isomere des Bisphenol-A zu Bisphenol-A isomerisiert;
(B) Inkontaktbringen von wenigstens einem Teil des resultierenden Abwassers aus (A) mit einem basischen Ionenaustauschharz, wodurch saure Verunreinigungen entfernt werden; und
(C) Abtrennen von Bisphenol-A aus dem besagten säurefreien Abwasser aus (B).

2. Verfahren nach Anspruch 1, worin teilchenförmige Materialien aus dem säurefreien Material durch Filtration entfernt werden.

3. Verfahren nach Anspruch 1, worin die Abtrennung durch Destillation erfolgt.

4. Verfahren nach Anspruch 3, worin die Destillation in Folgeschritten entfernt: (a) Phenol, (b) gefärbte leichte Fraktionen und (c) Bisphenol-A.

5. Verfahren zur Rückgewinnung von Bisphenol-A aus einer Mischung aus Bisphenol-A, Isomeren des Bisphenol-A, Phenol, sauren Verunreinigungen und verunreinigenden Nebenprodukten der Kondensationsreaktion von Aceton mit Phenol, welches umfaßt:
A. Isomerisierung der Isomeren von Bisphenol-A zu Bisphenol-A;
B. Entfernung der Säureverunreinigungen aus dem Isomerisat;
C. Filtrierung des besagten säurefreien Isomerisats zur Entfernung fester Verunreinigungen;
D. Destillation des teilchenfreien Materials zur Entfernung von Phenol;
E. Destillation des Bodenrückstandes (D) zur Entfernung einer gefärbten leichten Fraktion; und
F. Destillation der Bodenrückstände (E) zur Abtrennung des Bisphenol-A.

## Revendications

1. Procédé de récupération de bisphénol-A à partir d'une liqueur mère obtenue après cristallisation d'un produit d'addition 1:1 de phénol et de bisphénol-A, ladite liqueur mère contenant du phénol, du bisphénol-A, des isomères du bisphénol-A, des sous-produits contaminants provenant de la réaction de condensation du phénol avec l'acétone, et des impuretés acides, procédé qui comprend :
(A) la mise en contact d'au moins une partie de ladite liqueur mère avec un catalyseur qui provoque l'isomérisation des isomères du bisphénol-A en bisphénol-A,
(B) la mise en contact d'au moins une partie des effluents provenant de (A) avec une résine échangeuse d'ions basique, ce qui élimine les impuretés acides, et
(C) la séparation du bisphénol-A des effluents exempts d'acides, provenant de (B).

2. Procédé selon la revendication 1, dans lequel on élimine par filtration des particules du produit exempt d'acide.

3. Procédé selon la revendication 1, dans lequel la séparation est effectuée par distillation.

4. Procédé selon la revendication 3, dans lequel la distillation enlève, dans l'ordre, (a) du phénol, (b) une fraction légère colorée et (c) du bisphénol-A.

5. Procédé de récupération de bisphénol-A à partir d'un mélange de bisphénol-A, d'isomères de bisphénol-A, de phénol, d'impuretés acides et de sous-produits contaminants provenant de la réaction de condensation de l'acétone avec le phénol, qui comprend :
(A) l'isomérisation des isomères du bisphénol-A en bisphénol-A,
(B) l'élimination des produits contaminants acides des produits provenant de l'isomérisation,
(C) la filtration des produits d'isomérisation exempts d'acides, pour éliminer les particules solides,
(D) la distillation du produit exempt de particules solides, pour éliminer le phénol,
(E) la distillation des queues provenant de (D), pour éliminer une fraction légère colorée, et
(F) la distillation des queues provenant de (E), pour séparer le bisphénol-A.
